# EUROPEAN PATENT SPECIFICATION

(11) **EP 3 860 508 B1**
(45) Date of publication and mention of the grant of the patent: **31.07.2024**
(21) Application number: 19820880.3
(22) Date of filing: 06.12.2019
(51) Int. Cl.: A61F 2/16

(54) **BATTERY-POWERED INTRAOCULAR LENS INJECTOR**
BATTERIEBETRIEBENER INTRAOKULARLINSENINJEKTOR
INJECTEUR DE LENTILLE INTRAOCULAIRE FONCTIONNANT SUR BATTERIE

(30) Priority: 11.12.2018 US 201862777797 P
(43) Date of publication of application: 11.08.2021
(73) Proprietor: Alcon Inc., 1701 Fribourg (CH)
(72) Inventor: CHEN, Bill, Lake Forest, California 92630 (US); CHON, James Y., Lake Forest, California 92630 (US)
(74) Representative: Murgitroyd & Company
(86) International application number: PCT/IB2019/060522
(87) International publication number: WO 2020/121143

(56) References cited:
- WO-A1-2017/031037
- US-A- 4 836 201
- US-A1- 2008 097 460
- US-A1- 2013 197 532
- US-A1- 2017 027 686

## Description

### BACKGROUND

The human eye can suffer a number of maladies causing mild deterioration to complete loss of vision. While contact lenses and eyeglasses can compensate for some ailments, ophthalmic surgery may be required for others. Generally, ophthalmic surgery may be classified into posterior segment procedures, such as vitreoretinal surgery, and anterior segment procedures, such as cataract surgery. Vitreoretinal surgery may address many different eye conditions, including, but not limited to, macular degeneration, diabetic retinopathy, diabetic vitreous hemorrhage, macular hole, detached retina, epiretinal membrane, and cytomegalovirus retinitis.

For cataract surgery, a surgical procedure may require incisions and insertion of tools within an eye to replace a clouded natural lens with an intraocular lens (IOL). During this procedure, an opening is made in the anterior capsule and a thin phacoemulsification cutting tip may be inserted into the natural lens and vibrated ultrasonically. The vibrating cutting tip may liquefy or emulsify the natural lens so that it may be aspirated out of the eye. The natural lens, once removed, may be replaced by an IOL through the use of an IOL injector device. The IOL may be injected into the eye through the same small incision used to remove the natural lens. A nozzle tip of a pre-loaded insertion cartridge on the IOL injector may be inserted into the incision, and the IOL may be delivered into the eye.

Typically, IOLs may be manufactured from a polymer with specific characteristics. These characteristics allow the lens to be folded, and when delivered into the eye, allow the lens to unfold into the proper shape. Several manual injector devices are available for implanting these IOLs into the eye. However, threaded manual injectors require the use of two hands, which may be cumbersome and tedious. Syringe-type injectors may produce inconsistent injection force and displacement. Semi-automated injectors are also available, but require a cable that inhibits portability with an operator and may require a separate console for operation.

The relevant state of the art is represented by US 2013/197532 A1 and WO 2017/031037A1. Document US2008/0097460 discloses a device according to the preamble of claim 1.

### SUMMARY

The invention is defined by the device of independent claim 1. Any disclosed methods are merely exemplary and do not fall within the scope of the present invention. In an exemplary aspect, the present disclosure is directed to an IOL injector device. An example IOL injector device may include a housing, wherein the housing comprises a first end and a second end. The IOL injector device further may include a battery and a plunger disposed within the housing. The IOL injector device further may include a motor powered by the battery and configured to cause the plunger to translate in the housing. The IOL injector device further may include a user interface disposed on the housing, wherein the user interface receives user input to start translation of the plunger.

In another exemplary aspect, the present disclosure is directed to a method of operating an intraocular lens injector device. An example method of operating an intraocular lens injector device may include pushing a button disposed on a housing of the intraocular lens injector device to cause a battery to supply power to a motor disposed in the housing. The method may further include driving a plunger with the motor such that a tip of the plunger engages an intraocular lens held at an end of the housing. The method may further include displacing the intraocular lens into an eye with the plunger.

The different aspects may include one or more of the following features. The battery of the IOL injector device may be disposable. The batter of the IOL injector device may be rechargeable, wherein IOL injector device further comprises a charging station to receive the first end of the housing. The method may further include recharging the battery. The battery may be disposed on a printed circuit board. The intraocular lens injector device further includes a processor disposed on the printed circuit board, wherein the processor is configured to start translation of the motor in response to the user input. The printed circuit board may be disposed at the first end of the housing, wherein the motor is disposed beneath the printed circuit board. The motor may include a direct current brushless motor. The IOL injector device may further include a gear box, wherein the gear box is coupled to the motor, and wherein the gear box reduces angular velocity produced by the motor. The IOL injector device may further include an actuating unit, wherein the actuating unit is coupled to the gear box and the plunger, wherein the actuating unit converts rotary motion of the motor into linear motion. The IOL injector device may further include a cartridge mount disposed at the second end of the housing, wherein the cartridge mount is configured to hold an insertion cartridge in alignment with the plunger so that an intraocular lens disposed in the insertion cartridge is displaced from the insertion cartridge as the plunger is translated towards the second end. The plunger may include a plunger tip that is extendible through a hole at the second end of the housing to engage an intraocular lens disposed in a cartridge mount at the second end of the housing. The user interface includes a button and a display. The display is configured to display a string or text data type. The button and the display may be disposed at the second end of the housing. The button and the display are in signal communication with the processor on the circuit board on which the battery may be disposed. The method may further include displaying a status of the intraocular lens injector device to an operator with a display on the housing. The intraocular lens injector device may further include a printed circuit board disposed in the housing, a processor disposed on the printed circuit board in signal communication with the battery and configured to start translation of the motor in response to user input, one or more buttons on the housing in signal communication with the processor and configured to receiver the user input, and a display on the housing configured to display a string or text data type and in signal communication with the processor.

It is to be understood that both the foregoing general description and the following detailed description are exemplary and explanatory in nature and are intended to provide an understanding of the present disclosure without limiting the scope of the present disclosure. In that regard, additional aspects, features, and advantages of the present disclosure will be apparent to one skilled in the art from the following detailed description.

### BRIEF DESCRIPTION OF THE DRAWINGS

These drawings illustrate certain aspects of some of the embodiments of the present disclosure and should not be used to limit or define the disclosure.
FIG. 1 illustrates an example IOL injector device operable to deliver an IOL into an eye.
FIG. 2 illustrates an exploded view of the example IOL injector device of FIG. 1 with the housing detached to illustrate its internal components.
FIGs. 3A and 3B illustrate an example IOL injector device with a charging station.
FIG. 4 illustrates a block diagram of an example IOL injector device that can be used in treatment of a patient.
FIG. 5A illustrates an eye in which an IOL is being inserted from an example IOL injector device.
FIG. 5B illustrates the eye shown in FIG. 5A in which the IOL is positioned within the capsulate bag of the eye and the IOL injector device is removed from the eye.

### DETAILED DESCRIPTION

For the purposes of promoting an understanding of the principles of the present disclosure, reference will now be made to the implementations illustrated in the drawings and specific language will be used to describe them. It will nevertheless be understood that no limitation of the scope of the disclosure may be intended. Any alterations and further modifications to the described devices, instruments, methods, and any further application of the principles of the present disclosure are fully contemplated as would normally occur to one skilled in the art to which the disclosure relates. In particular, it is fully contemplated that the features, components, and/or steps described with reference to one or more implementations may be combined with the features, components, and/or steps described with reference to other implementations of the present disclosure. For simplicity, in some instances the same reference numbers may be used throughout the drawings to refer to the same or like parts.

The example embodiments described herein generally relate to eye surgery. More particularly, the example embodiments generally relate to systems, methods, and devices for inserting an intraocular lens (IOL) into an eye. Embodiments include an IOL injector device that is battery powered and may include a plunger and an insertion cartridge. In some embodiments, the plunger may force the IOL through the insertion cartridge and out a tip of a nozzle of the insertion cartridge. By being battery powered, the IOL injector device may not need a console and/or footswitch, thus having increased portability over current cabled IOL injectors. Additionally, the battery power automates delivery of the IOL, reducing the complexity associated with current manual IOL injector devices that can require multiple hands and consistent injection force.

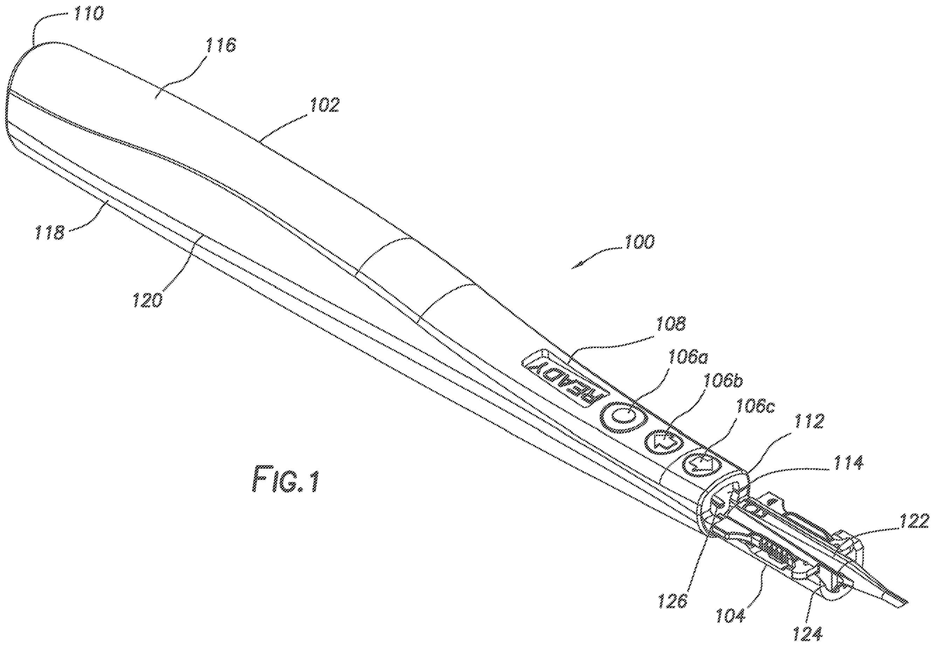

FIG. 1 illustrates an example IOL injector device 100. In some embodiments, the IOL injector device 100 includes a housing 102, a cartridge mount 104, one or more buttons 106, and a display 108. The housing 102 may be any suitable size, height, and/or shape. Without limitation, a suitable shape may include, but is not limited to, cross-sectional shapes that are circular, elliptical, triangular, rectangular, square, hexagonal, and/or combinations thereof. In some embodiments, the housing 102 has a circular cross-sectional shape and may be a cylinder of a specified length and radius suitable for grasping by an operator's hand. The housing 102 may be made from any suitable material. Suitable materials may include, but are not limited to, metals, nonmetals, polymers, ceramics, and/or combinations thereof. In some embodiments, the housing 102 may have a first portion 116 and a second portion 118. In some embodiments, the first portion 116 and the second portion 118 may join along a longitudinally extending interface 120. In the illustrated embodiment, the housing 102 has a first end 110 and a second end 112. An operator of the IOL injector device 100 may hold the IOL injector device 100 about the first end 110. The second end 112 may include a hole 114 that allows access to a central bore (obstructed from view) of the housing 102. In some embodiments, the cartridge mount 104 may be disposed at the second end 112 of the housing 102.

The cartridge mount 104 may be disposed at the second end 112 of the housing 102, by using any suitable mechanism, including, but not limited, through the use of suitable fasteners, threading, adhesives, snap-fit methods, welding, and/or any combination thereof. Without limitation, suitable fasteners may include nuts and bolts, washers, screws, pins, sockets, rods and studs, hinges and/or any combination thereof. In the illustrated embodiment, the cartridge mount 104 is extension from the housing 102. In some instances, the cartridge mount 104 may be integrally connected to the housing 102. In other instances, the cartridge mount 104 may be separate from the housing 102 and may be coupled to the housing 102 via an interlocking relationship. As depicted in FIG. 1, the cartridge mount 104 holds a removably mounted insertion cartridge 122. In some instances, the cartridge mount 104 includes a channel 124 that may be a unique cutout (e.g., u-shaped) to accommodate the insertion cartridge 122. In alternate embodiments, the cartridge mount 104 may be any suitable size, height, and/or shape. In some embodiments, the insertion cartridge 122 may be a disposable polymeric component adapted to accommodate an unfolded IOL and to fold and displace the IOL as a plunger tip 126 is translated forward from the housing 102 and through the insertion cartridge 122.

The one or more one or more buttons 106a-106ca-106c may be disposed at any suitable location on the housing 102. The one or more buttons 106a-106c are referred to herein collectively as one or more buttons 106a-106c and individually as first one or more buttons 106a-106ca, second one or more buttons 106a-106cb, and third one or more buttons 106a-106cc. In some embodiments, the one or more buttons 106 may be disposed at or near the second end, as shown on FIG. 1. In some embodiments, the one or more buttons 106a-106c proximate to the display 108. While three of the one or more buttons 106a-106c are shown on FIG. 1, there may be more or less than three of the one or more buttons 106a-106c, as desired for a particular application. The one or more buttons 106a-106c may be any suitable size, height, and/or shape. Without limitation, a suitable shape may include, but is not limited to, cross-sectional shapes that are circular, elliptical, triangular, rectangular, square, hexagonal, and/or combinations thereof. In some embodiments, the one or more buttons 106a-106c may have a circular cross-sectional shape and may resemble a commonplace "power button." The one or more buttons 106a-106c may be made from any suitable material. Suitable materials may include, but are not limited to, metals, nonmetals, polymers, ceramics, and/or combinations thereof.

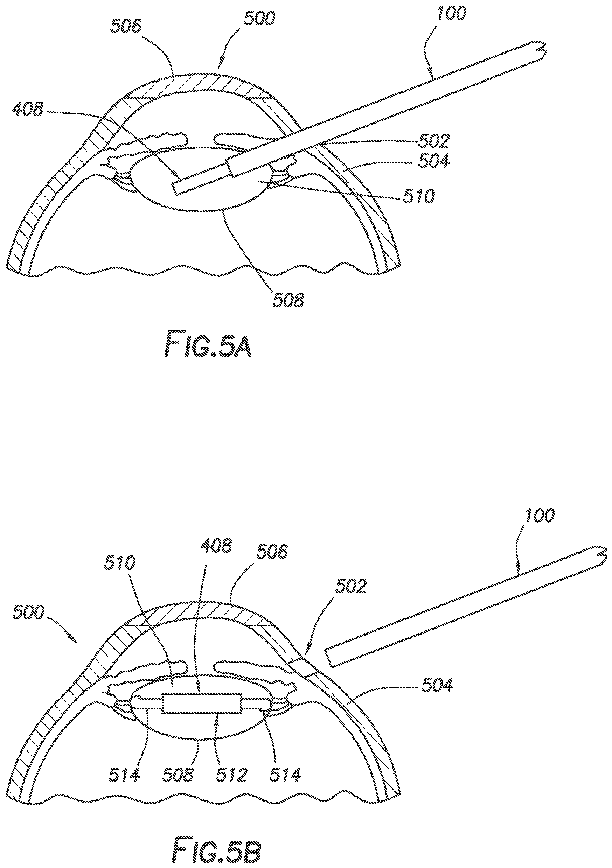

In some embodiments, the one or more buttons 106a-106c may be used to trigger one or more different events. For example, the first button 106a may be an on/off switch. By way of further example, the third button 106c may trigger the insertion process to start while the second button 106b may trigger the plunger tip 126 to retract. In some embodiments, each of the one or more buttons 106a-106c may be triggered to trigger more than one event. In some embodiments, an operator may turn on the IOL injector device 100 by pushing down on the first button. Next, embodiments may include actuating the IOL injector device 100 by pushing down on the third button 106c. In some embodiments, actuating the IOL injector device 100 may translate the plunger tip 126 through the insertion cartridge 122 in order to displace the IOL (e.g., IOL 408 shown on FIG. 5A) from the insertion cartridge 122. In some embodiments, pushing the second button 106b may stop translation of the plunger tip 126 with yet another push of the second button 106b causing retraction of the plunger tip 126. In addition, while FIG. 1 illustrates one or more buttons 106a-106c, it should be understood that other suitable user interfaces may be incorporated into the IOL injector device 100 in addition to, or in place of the one or more buttons 106a-106c, including but not limited to, one or more switches or a touch screen display.

The display 108 may be any suitable device for presenting visual information to the operator of the status of the IOL injector device 100. As shown on FIG. 1, the display 108 can be in the form of a screen positioned on the housing 102 whereupon a string and/or text data type indication may be displayed to an operator. Any suitable indication may be shown by the display 108. Without limitation, example indications may include "Ready," "Error," "Retract," "Charged," or "Paused." The display 108 may be any suitable size, height, and/or shape. In the illustrated embodiment, the display 108 is rectangular. While FIG. 1 illustrates a display 108, it should be understood that other suitable operator indicators may be used for providing information to the operator, including, but not limited to, speakers for providing audible status indicators or lights for providing visual or color coded status indicators.

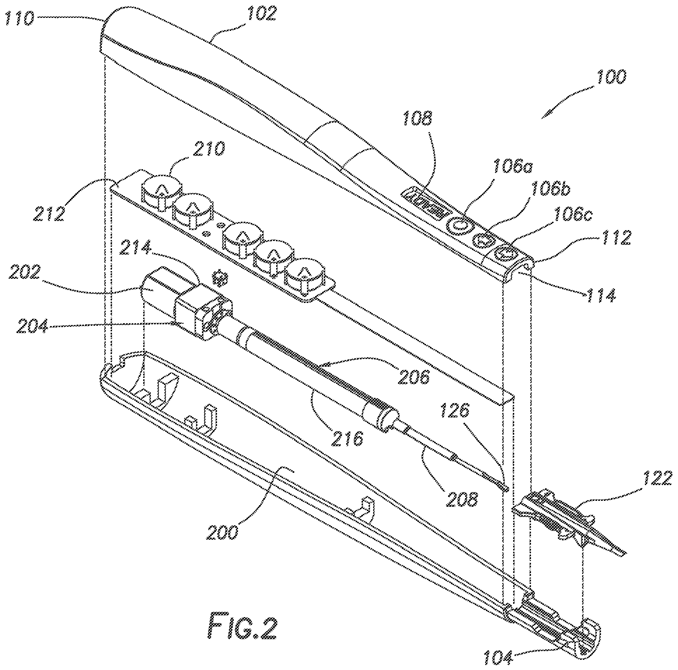

FIG. 2 illustrates an exploded view of the example IOL injector device 100 of FIG. 1. Example embodiments of the housing 102 include an internal cavity 200. As illustrated, the IOL injector device 100 includes a motor 202, a gear box 204, an actuating unit 206, a plunger 208, one or more batteries 210, and a circuit board 212, each of which are shown disposed in the internal cavity 200.

The motor 202 may be any suitable electric motor capable of converting electrical energy from the one or more batteries 210 into mechanical energy. In some embodiments, the motor 202 provides rotational torque. However, it is also contemplated that the motor 202 may convert the electrical energy from the one or more batteries 210 into linear motion. Without limitation, the motor 202 may be a direct current (DC) brushless motor, DC brush motor, stepper motor, and/or an induction motor. The motor 202 may have any suitable dimensions to remain disposed within the internal cavity 200 of the IOL injector device 100. In some embodiments, the motor 202 may be disposed at the first end 110 of the IOL injector device 100 in the internal cavity 200 of the housing 102.

In some embodiments, the gear box 204 is coupled to the motor 202. As illustrated, the gear box 204 is disposed in the internal cavity 200 adjacent to the motor 202 at the first end of the IOL injector device 100. The gear box 204 may include a gear housing 214 and any suitable number of gears (not shown) disposed within the gear housing 214 (best seen on FIG. 2). In some embodiments, the gear box 204 may reduce the angular velocity of the motor 202 according to a specified gear ratio. The gear ratio may be any suitable ratio between the rates at which the first and last gear rotates. Examples of suitable gear ratios may include, but are not limited to, a gear ratio ranging from about 125:1 to about 250:1. In some embodiments, the implementation of the gear box 204 may increase the available torque from the motor 202 and reduce the speed at which the actuating unit 206 translates.

In some embodiments, the actuating unit 206 may convert rotary motion from the motor 202 into linear motion. In the illustrated embodiment, the actuating unit 206 is disposed within internal cavity 200 of the housing 102 adjacent to the gear box 204, wherein the actuating unit 206 is coupled to the gear box 204. The gear box 204 may be disposed between the actuating unit 206 and the motor 202. The actuating unit 206 may be any suitable size, height, and/or shape. In some embodiments, any suitable linear actuator may be used with the actuating unit 206 for conversion of the rotary motion to linear motion. Without limitations, the actuating unit 206 may include any suitable linear actuator, including, but not limited to, a leadscrew actuator, screw jack actuator, ball screw actuator, and/or roller screw actuator. In some embodiments, the actuating unit 206 includes an outer housing 216. For example, the outer housing 216 may be a hollow tubular in which the linear actuator (not illustrated) may be disposed.

In some embodiments, the plunger 208 is coupled to the actuating unit 206. For example, the plunger may be coupled to the linear actuator (not illustrated) disposed within the outer housing 216. The plunger 208 may be affixed to the actuating unit 206 by using any suitable mechanism, including, but not limited, the use of suitable fasteners, threading, adhesives, snap-fit methods, welding, and/or any combination thereof. Examples of suitable fasteners may include, but are not limited to, nuts and bolts, washers, screws, pins, sockets, rods and studs, hinges and/or any combination thereof. As the actuating unit 206 operates, in some embodiments, the plunger 208 is displaced in a linear fashion. As the linear actuator disposed within the actuating unit 206 rotates due to the rotational motion provided by the motor 202, the plunger 208 can translate linearly. As the plunger 208 translates through the internal cavity 200 of the housing 102, the plunger tip 126 may extend through the second end 112 of the housing 102. For example, the plunger tip 126 extends through hole 114 in the second end 112 to engage the insertion cartridge 122 disposed on the cartridge mount 104.

In some embodiments, the IOL injector device 100 includes one or more batteries 210 disposed within the housing 102 of the IOL injector device 100. For example, the one or more batteries 210 are disposed on the circuit board 212 in the internal cavity 200 of the housing 102. In some embodiments, the one or more batteries 210 supply power to the motor 202 in order to operate the IOL injector device 100. It should be understood that while the present example shows only five of the one or more batteries 210, the examples embodiments are intended to encompass any number of batteries 210 suitable for providing power to the IOL injector device 100. The one or more batteries 210 may be disposable and/or rechargeable. The one or more batteries 210 may include any suitable type of battery, including, but not limited to, alkaline 1.5 V batteries. In some embodiments, the minimum voltage needed to operate the IOL injector device 100 may be about 7.5 V. In some embodiments, the one or more batteries 210 may be any suitable size, height, and/or shape. Without limitation, a suitable shape may include, but is not limited to, cross-sectional shapes that are circular, elliptical, triangular, rectangular, square, hexagonal, and/or combinations thereof. As depicted, the plurality of batteries 210 may be circular in cross-section. In alternate embodiments, each battery 210 may be a different shape.

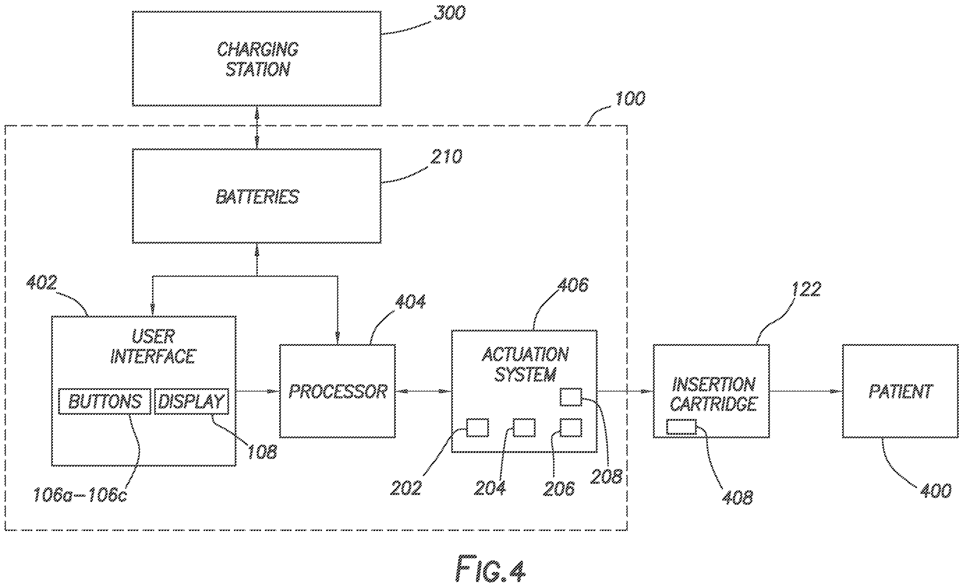

The circuit board 212 may be any suitable circuit board, including, but not limited to, a printed circuit board. In some embodiments, the plurality of batteries 210 may be soldered onto the circuit board 212 along with any other suitable electronic component (e.g., processor 404 shown on FIG. 4). In some embodiments, the circuit board 212 may be disposed at the first end 110 of the housing 102 of the IOL injector device 100 and extend towards the second end 112. The circuit board 212 may span the length of the motor 202, the gear box 204, and/or a portion of the actuating unit 206. In some embodiments the circuit board 212 and/or the plurality of batteries 210 may be wired to, or otherwise in signal communication with, the motor 202, the one or more buttons 106a-106c, the display 108, and/or combinations thereof.

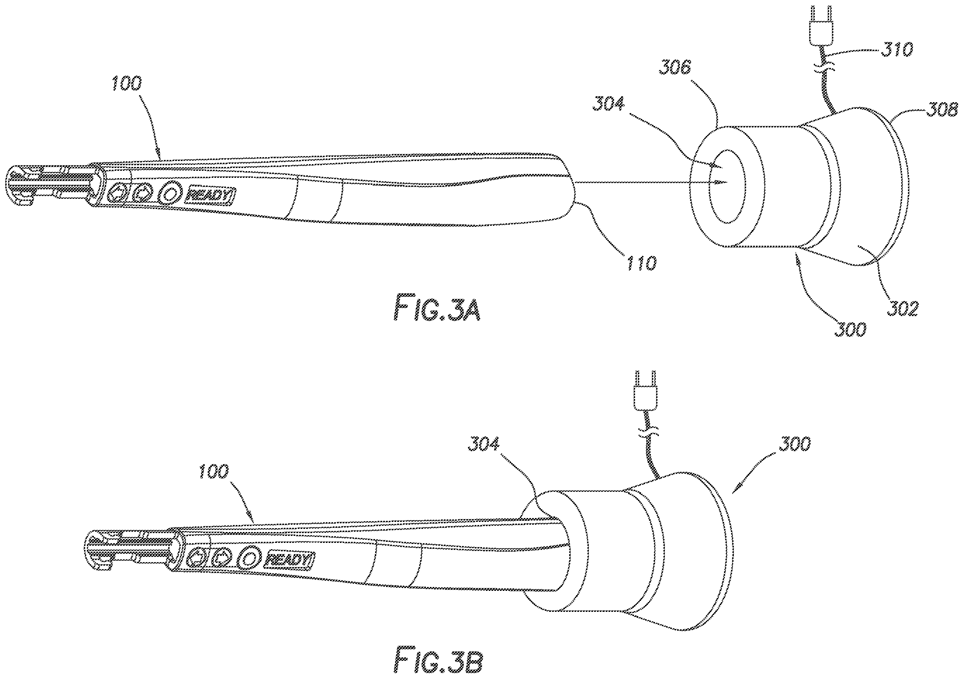

FIG. 3A illustrates an embodiment of the IOL injector device 100 with a charging station 300. The charging station 300 may be utilized to charge the one or more batteries 210 (e.g., referring to FIG. 2) in the example embodiments where one or more batteries 210 are rechargeable. In some embodiments, the charging station 300 may be any suitable size, height, and/or shape. Without limitation, a suitable shape may include, but is not limited to, cross-sectional shapes that are circular, elliptical, triangular, rectangular, square, hexagonal, and/or combinations thereof. While not illustrated, the charging station 300 may be in the form of a flat pad, in some embodiments. In the illustrated embodiment, the charging station 300 includes a charger body 302 and a hole 304. The hole 304 may be disposed at a first end 306 of the charger body 302. The hole 304 may be configured to receive the first end 110 of the IOL injector device 100. A second end 308 of the charger body 302 may be flat whereupon the charger body 302 may lay at rest. An electrical cable 310 may be coupled to the charger body 302. The electrical cable 310 may couple the charger body 302 to a power source (not shown) for recharging the IOL injector device 100. With reference now to FIG. 3B, In some embodiments, the IOL injector device 100 may be inserted into the hole 304 in order to charge the one or more batteries 210. It may take any suitable amount of time to fully charge the one or more batteries 210. It should be understood that the charging station 300 may use any suitable technique for recharging the one or more batteries. For example, the charging station 300 may use a wireless charging technique, such as inductive charging, radio charging, or resonance charging.

FIG. 4 is a block diagram illustrating certain components of an IOL injector device 100 for treatment of a patient 400. As illustrated, the example IOL injector device 100 includes one or more batteries 210, user interface 402, processor 404, and actuation system 406. In some embodiments, the one or more batteries 210 may power the other components of the IOL injector device 100, including, but not limited, the user interface 402, the processor 404, and/or the actuation system 406. For example, the one or more batteries 210 provide power to the motor 202 of the actuation system 406. In the illustrated embodiment, the user interface 402 includes one or more buttons 106-106c and display 108. In some embodiments, where the one or more batteries 210 are rechargeable, charging station 300 may be used, for example, to charge the one or more batteries 210 prior to use. The user interface 402 may receive input from an operator, for example, by way of the one or more buttons 106a-106c, and may provide information to an operator, for example, by way of the display 108. The processor 404 may be configured to receive inputs from an operator by way of the user interface 402, for example, to start and/or to stop the operation the IOL injector device 100. The processor 404 is disposed on the circuit board 212 (e.g., shown on FIG. 2). The processor 404 may include any suitable device for processing instructions, including, but not limited to, a microprocessor, microcontroller, embedded microcontroller, programmable digital signal processor, or other programmable device. The processor 404 may also, or instead, be embodied in an application specific integrated circuit, a programmable gate array, programmable array logic, or any other device of combinations of devices operable to process electric signals. The processor 404 may be communicatively coupled to user interface 402 and the actuation system 406. In some embodiments, the actuation system 406 includes motor 202, gear box 204, actuating unit 206, and plunger 208. The actuation system 406 may be configured to receive commands from the processor 404. For example, the processor 404 may instruct the motor 202 to operate. The actuating unit 206 may convert the rotary motion from the motor 202 into linear motion. As the actuating unit 206 displaces the plunger 208, the plunger tip 126 (e.g., shown on FIG. 1) may engage the insertion cartridge 122. For example, the plunger 208 may push against an IOL 408 disposed within the insertion cartridge 122 and cause the IOL 408 to displace through and/or out of the insertion cartridge 122 into an eye (e.g., eye 500 shown on FIG. 5A) of the patient 400. The IOL 408 may have a shape similar to that of a natural lens of the eye. The IOL 408 may be made from numerous materials, including, but not limited to, silicone, acrylic, and/or combinations thereof. Other suitable materials are also contemplated.

FIG. 5A illustrates an eye 500 of a patient undergoing an operation with IOL injector device 100. As illustrated, the IOL injector device 100 dispenses an IOL 408 into the eye 500 of a patient. In some embodiments, the IOL 408 may be in a folded state. In some embodiments, an incision 502 is made in the eye 500 by a surgeon. For example, in some instances, the incision 502 may be made through the sclera 504 of the eye 500. In other instances, the incision 502 may be formed in the cornea 506 of the eye 500. The incision 502 may be sized to permit insertion of a portion of the IOL injector device 100 in order to deliver the IOL 408 into the capsular bag 508. For example, in some instances, the size of the incision 502 may have a length less than about 2000 microns (2 millimeters). In other instances, the incision 502 may have a length from about 0 microns to about 500 microns; from about 500 microns to about 1000 microns; from about 1000 microns to about 1500 microns; or from about 1500 microns to about 2000 microns.

After the incision 502 is made, the IOL injector device 100 is inserted through the incision 502 into an interior portion 510 of the eye 500. The IOL injector device 100 is actuated to dispense the IOL 408 into the capsular bag 508 of the eye 500. Upon dispensation, the IOL 408 may revert to an initial, unfolded state, and the IOL 408 settles within the capsular bag 508 of the eye 500, as shown on FIG. 5B. The capsular bag 508 holds the IOL 408 within the eye 500 in a relationship relative to the eye 500 so that an optic 512 of the IOL 408 refracts light directed to the retina (not shown). The IOL 408 includes haptics 514 that extend from the optic 512 and engage the capsular bag 508 to secure the IOL 408 therein. After dispensing the IOL 408 into the capsular bag 508, the IOL injector device 100 is removed from the eye 500 through the incision 502, and the eye 500 is allowed to heal over time.

It is believed that the operation and construction of the present disclosure will be apparent from the foregoing description. While the apparatus and methods shown or described above have been characterized as being preferred, various changes and modifications may be made therein without departing from the scope of the invention as defined by the following claims.

## Claims

1. An intraocular lens injector device (100), comprising:
a housing (102), wherein the housing comprises a first end (110) and a second end (112);
a battery (210);
a plunger (208) disposed within the housing;
a motor (202) powered by the battery and configured to cause the plunger to translate in the housing;
a user interface (402) disposed on the housing, wherein the user interface receives user input to start translation of the plunger; and
**characterised by**
a printed circuit board (212) disposed in the housing, a processor (404) disposed on the printed circuit board in signal communication with the battery and configured to start translation of the motor in response to user input, one or more buttons (106) on the housing in signal communication with the processor and configured to receive the user input, and a display (108) on the housing configured to display a string or text data type and in signal communication with the processor.

2. The intraocular lens injector device (100) of claim 1, wherein the battery (210) is rechargeable, wherein intraocular lens injector device further comprises a charging station (300) to receive the first end (110) of the housing (102).

3. The intraocular lens injector device (100) of claim 1, wherein the battery (210) is disposed on the printed circuit board.

4. The intraocular lens injector device (100) of claim 3, wherein the printed circuit board (212) is disposed at the first end (110) of the housing (102), wherein the motor (202) is disposed beneath the printed circuit board.

5. The intraocular lens injector device (100) of claim 1, wherein the motor (202) comprises a direct current brushless motor.

6. The intraocular lens injector device (100) of claim 1, further comprising a gear box (204), wherein the gear box is coupled to the motor (202), and wherein the gear box reduces angular velocity produced by the motor.

7. The intraocular lens injector device (100) of claim 6, further comprising an actuating unit (206), wherein the actuating unit is coupled to the gear box (204) and the plunger (208), wherein the actuating unit converts rotary motion of the motor (202) into linear motion.

8. The intraocular lens injector device (100) of claim 1, further a cartridge mount (104) disposed at the second end (112) of the housing (102), wherein the cartridge mount is configured to hold an insertion cartridge (122) in alignment with the plunger (208) so that an intraocular lens disposed in the insertion cartridge is displaced from the insertion cartridge as the plunger is translated towards the second end.

9. The intraocular lens injector device (100) of claim 1, wherein the plunger (208) comprises a plunger tip (126) that is extendible through a hole at the second end (112) of the housing (102) to engage an intraocular lens disposed in a cartridge mount (104) at the second end of the housing.

10. intraocular lens injector device (100) of claim 1, wherein the one or more buttons (106) and the display are disposed at the second end (112) of the housing (102).

## Patentansprüche

1. Intraokularlinseninjektorvorrichtung (100), umfassend:
ein Gehäuse (102), wobei das Gehäuse ein erstes Ende (110) und ein zweites Ende (112) umfasst;
eine Batterie (210);
einen Kolben (208), der in dem Gehäuse angeordnet ist;
einen Motor (202), der von der Batterie angetrieben wird und ausgestaltet ist, um Translatieren des Kolbens in dem Gehäuse zu bewirken;
eine Benutzerschnittstelle (402), die auf dem Gehäuse angeordnet ist, wobei die Benutzerschnittstelle Benutzereingabe empfängt, um mit der Translation des Kolbens zu beginnen; und
**gekennzeichnet durch**
eine gedruckte Schaltplatine (212), die in dem Gehäuse angeordnet ist, einen Prozessor (404), der auf der gedruckten Schaltplatine in Signalkommunikation mit der Batterie angeordnet ist und ausgestaltet ist, um in Reaktion auf Benutzereingabe mit der Translation des Motors zu beginnen, eine oder mehrere Schaltflächen (106) auf dem Gehäuse in Signalkommunikation mit dem Prozessor und ausgestaltet, um Benutzereingabe zu empfangen, und eine Anzeige (108) auf dem Gehäuse, die ausgestaltet ist, um einen Zeichenketten- oder Textdatentyp anzuzeigen, und in Signalkommunikation mit dem Prozessor.

2. Intraokularlinseninjektorvorrichtung (100) nach Anspruch 1, wobei die Batterie (210) aufladbar ist, wobei die Intraokularlinseninjektorvorrichtung des Weiteren eine Ladestation (300) umfasst, um das erste Ende (110) des Gehäuses (102) anzunehmen.

3. Intraokularlinseninjektorvorrichtung (100) nach Anspruch 1, wobei die Batterie (210) auf der gedruckten Schaltplatine angeordnet ist.

4. Intraokularlinseninjektorvorrichtung (100) nach Anspruch 3, wobei die gedruckte Schaltplatine (212) an dem ersten Ende (110) des Gehäuses (102) angeordnet ist, wobei der Motor (202) unter der gedruckten Schaltplatine angeordnet ist.

5. Intraokularlinseninjektorvorrichtung (100) nach Anspruch 1, wobei der Motor (202) einen bürstenlosen Gleichstrommotor umfasst.

6. Intraokularlinseninjektorvorrichtung (100) nach Anspruch 1, des Weiteren umfassend ein Schaltgetriebe (204), wobei das Schaltgetriebe an den Motor (202) gekoppelt ist, und wobei das Schaltgetriebe die durch den Motor produzierte Winkelgeschwindigkeit reduziert.

7. Intraokularlinseninjektorvorrichtung (100) nach Anspruch 6, des Weiteren umfassend eine Aktuatoreinheit (206), wobei die Aktuatoreinheit an das Schaltgetriebe (204) und den Kolben (208) gekoppelt ist, wobei die Aktuatoreinheit Drehbewegung des Motors (202) in Linearbewegung konvertiert.

8. Intraokularlinseninjektorvorrichtung (100) nach Anspruch 1, des Weiteren umfassend eine Patronenhalterung (104), die am zweiten Ende (112) des Gehäuses (102) angeordnet ist, wobei die Patronenhalterung ausgestaltet ist, um eine Einführpatrone (122) in Ausrichtung mit dem Kolben (208) zu halten, so dass eine in der Einführpatrone angeordnete Intraokularlinse aus der Einführpatrone herausgeschoben wird, wenn der Kolben in Richtung des zweiten Endes translatiert wird.

9. Intraokularlinseninjektorvorrichtung (100) nach Anspruch 1, wobei der Kolben (208) eine Kolbenspitze (126) umfasst, die durch ein Loch an dem zweiten Ende (112) des Gehäuses (102) ausfahrbar ist, um in Eingriff mit einer Intraokularlinse zu kommen, die in einer Patronenhalterung (104) am zweiten Ende des Gehäuses angeordnet ist.

10. Intraokularlinseninjektorvorrichtung (100) nach Anspruch 1, wobei die eine oder mehreren Schaltflächen (106) und die Anzeige am zweiten Ende (112) des Gehäuses (102) angeordnet sind.

## Revendications

1. Dispositif injecteur de lentille intraoculaire (100), comprenant :
un boîtier (102), le boîtier comprenant une première extrémité (110) et une seconde extrémité (112) ;
une batterie (210) ;
un piston (208) disposé à l'intérieur du boîtier ;
un moteur (202) alimenté par la batterie et configuré pour amener le piston à se déplacer en translation dans le boîtier ;
une interface utilisateur (402) disposée sur le boîtier, l'interface utilisateur recevant une entrée de l'utilisateur pour démarrer la translation du piston ; et
**caractérisé par**
une carte de circuit imprimé (212) disposée dans le boîtier, un processeur (404) disposé sur la carte de circuit imprimé en communication de signal avec la batterie et configuré pour démarrer la translation du moteur en réponse à l'entrée de l'utilisateur, un ou plusieurs boutons (106) sur le boîtier en communication de signal avec le processeur et configurés pour recevoir l'entrée de l'utilisateur, et un dispositif d'affichage (108) sur le boîtier configuré pour afficher une chaîne de caractères ou un type de données textuelles et en communication de signal avec le processeur.

2. Dispositif injecteur de lentille intraoculaire (100) selon la revendication 1, la batterie (210) étant rechargeable, le dispositif injecteur de lentille intraoculaire comprenant en outre une station de charge (300) pour recevoir la première extrémité (110) du boîtier (102).

3. Dispositif injecteur de lentille intraoculaire (100) selon la revendication 1, la batterie (210) étant disposée sur la carte de circuit imprimé.

4. Dispositif injecteur de lentille intraoculaire (100) selon la revendication 3, la carte de circuit imprimé (212) étant disposée à la première extrémité (110) du boîtier (102), le moteur (202) étant disposé sous la carte de circuit imprimé.

5. Dispositif injecteur de lentille intraoculaire (100) selon la revendication 1, le moteur (202) comprenant un moteur sans balai à courant continu.

6. Dispositif d'injecteur de lentille intraoculaire (100) selon la revendication 1, comprenant en outre une boîte d'engrenages (204), la boîte d'engrenages étant couplée au moteur (202), et la boîte d'engrenages réduisant la vitesse angulaire produite par le moteur.

7. Dispositif injecteur de lentille intraoculaire (100) selon la revendication 6, comprenant en outre une unité d'actionnement (206), l'unité d'actionnement étant couplée à la boîte d'engrenages (204) et au piston (208), l'unité d'actionnement convertissant le mouvement rotatif du moteur (202) en mouvement linéaire.

8. Dispositif injecteur de lentille intraoculaire (100) selon la revendication 1, en outre un support de cartouche (104) disposé à la seconde extrémité (112) du boîtier (102), le support de cartouche étant configuré pour maintenir une cartouche d'insertion (122) dans l'alignement du piston (208) de sorte qu'une lentille intraoculaire disposée dans la cartouche d'insertion est déplacée de la cartouche d'insertion lorsque le piston est translaté vers la seconde extrémité.

9. Dispositif injecteur de lentille intraoculaire (100) selon la revendication 1, le piston (208) comprenant une pointe de piston (126) qui est extensible à travers un trou à la seconde extrémité (112) du boîtier (102) pour venir en prise avec une lentille intraoculaire disposée dans une monture de cartouche (104) à la seconde extrémité du boîtier.

10. Dispositif injecteur de lentille intraoculaire (100) selon la revendication 1, le ou les boutons (106) et l'affichage étant disposés à la seconde extrémité (112) du boîtier (102).
